(19) Europäisches Patentamt
European Patent Office
Office européen des brevets

(11) Veröffentlichungsnummer : **0 120 290**
**B1**

(12) **EUROPÄISCHE PATENTSCHRIFT**

(45) Veröffentlichungstag der Patentschrift : **19.08.87**

(51) Int. Cl.[4] : **C 07 D307/94**, C 11 B 9/00

(21) Anmeldenummer : **84101820.3**

(22) Anmeldetag : **22.02.84**

(54) **Oxaspirododecan-Derivate, deren Herstellung und Verwendung als Riechstoffe sowie diese enthaltende Riechstoffkompositionen.**

(30) Priorität : **26.02.83 DE 3306798**

(43) Veröffentlichungstag der Anmeldung : **03.10.84 Patentblatt 84/40**

(45) Bekanntmachung des Hinweises auf die Patenterteilung : **19.08.87 Patentblatt 87/34**

(84) Benannte Vertragsstaaten : **CH DE FR GB LI NL**

(56) Entgegenhaltungen :
**US-A- 4 009 127**
**CHEMICAL ABSTRACTS, REGISTRY HANDBOOK, Number Section, 1977, Supplement, Columbus, Ohio, USA**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Schaper, Armin Ulf, Dr.**
**Randstrasse 18a**
**D-4150 Krefeld (DE)**
Erfinder : **Blösl, Siegfried, Dr.**
**Itterstrasse 33**
**D-4000 Düsseldorf 13 (DE)**
Erfinder : **Bruns, Klaus, Prof. Dr.**
**Notburgaweg 6**
**D-4150 Krefeld-Traar (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Wegen der ungleichmäßigen Verfügbarkeit vieler natürlicher Riechstoffkomponenten und der notwendigen Anpassung an wechselnde modische Geschmacksrichtungen hat die Riechstoffindustrie ständig Bedarf an neuen Riechstoffen, die entweder allein oder in Form von Kompositionen wertvolle Parfums mit interessanten Duftnoten darstellen. Da aufgrund wenig bekannter Zusammenhänge zwischen Struktur und Riechstoffeigenschaften die gezielte Synthese von Riechstoffen mit gewünschter olfaktorischer Qualität nicht möglich ist, besteht die Aufgabe, solche Verbindungen aufzufinden, die wertvolle Riechstoffeigenschaften aufweisen.

Es wurde nun gefunden, daß 1-Oxaspiro-[4,7]-dodecane der Formeln (1) oder (2)

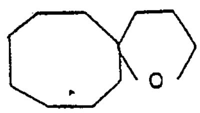
oder
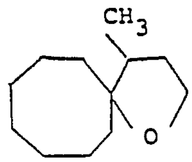

(1) (2)

ausgezeichnete neue Riechstoffe sind. Die Verbindungen sind relativ leicht flüchtig und daher besonders geruchsstark.

Besonders interessant sind die Geruchseigenschaften der Verbindung (1) mit einer Zibet-, Skatol-, Indol-Note. Aber auch die Verbindung (2) mit einer erdig-, technisch-, indolartigen Note kann für bestimmte Riechstoffkompositionen vorteilhaft verwendet werden.

Die Substanzen sind bisher nicht bekannt. Ihre Herstellung gelingt nach literaturbekannten Methoden nach folgendem Schema :

Im ersten Reaktionsschritt erfolgt radikalische Addition von Acrylsäure an Cyclooctanol. Die Reaktion wird durch Di-tert.-butylperoxid gestartet. Die intermediär entstehende Hydroxysäure cyclisiert unter den Reaktionsbedingungen sofort zum Lacton (3) :

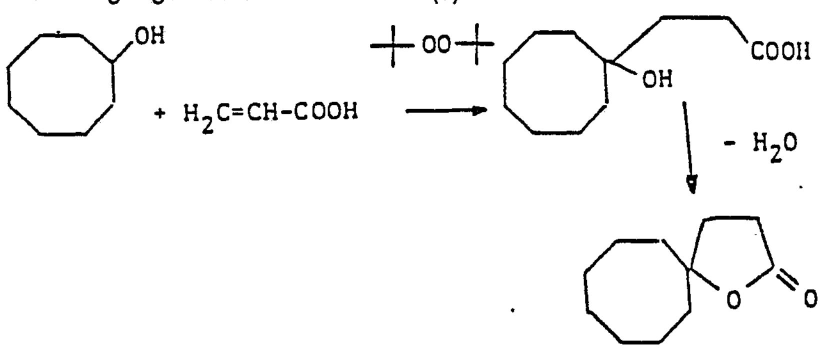


(3)

Um rasches Polymerisieren der Acrylsäure zu vermeiden, muß in verdünnter Lösung gearbeitet werden, d. h., ein 5-20-molarer Überschuß des Cyclooctanols ist zweckmäßig. Anschließend wird das Lacton (3) mit einem komplexen Metallhydrid, wie z. B. $LiAlH_4$, in sehr guter Ausbeute zum Diol (4) reduziert :

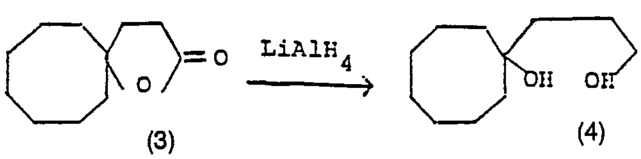


(3) (4)

Das Diol wird nicht isoliert. Im dritten Verfahrenschritt erfolgt durch azeotropes Abdestillieren des Wassers mit Toluol die Cyclisierung zum erfindungsgemäßen 1-Oxa-spiro-[4,7]-dodecan (1). Als Katalysator dient para-Toluolsulfonsäure in einer Menge von ca. 10 %, bezogen auf Diol, da mit geringeren Mengen bevorzugt der ungesättigte Alkohol gebildet wird.

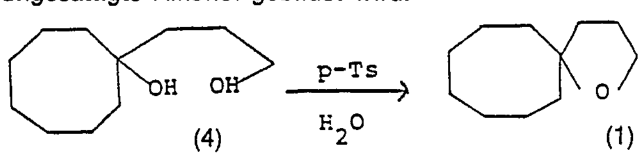


(4) (1)

Die Darstellung von 1-Oxa-4-methyl-spiro-[4.7]-dodecan (2) wird analog mit Crotonsäure (statt Acrylsäure) durchgeführt.

In Verbindung mit anderen Riechstoffen und gegebenenfalls gängigen Parfümbestandteilen lassen sich die neuen Verbindungen zu neuen, interessanten Riechstoffkompositionen kombinieren. Dabei werden etwa 1-50 Gew.-% der neuen Verbindungen, bezogen auf die gesamte Komposition, eingesetzt. Derartige Kompositionen können zur Parfümierung von Kosmetika, wie Duftwässern, Cremes, Lotionen, Aerosolen, Toilettenseifen, in der Extrait-Parfümerie, sowie zur Geruchsverbesserung von technischen Artikeln, wie Wasch- und Reinigungsmitteln, Desinfektionsmitteln, Textilbehandlungsmitteln und dergleichen dienen. Zur Parfümierung der verschiedenen Produkte werden diesen die Kompositionen in Mengen von 0.05-2 Gew.-%, bezogen auf das gesamte Produkt, zugesetzt.

## Beispiele

### A. Herstellung der Lactone

3 mol Cyclooctanol und 0,045 mol Di-tert.-butylperoxid wurden vorgelegt und auf 150 °C erhitzt. 0,3 mol Acrylsäure bzw. Crotonsäure wurden in 1 mol Cyclooctanol gelöst und unter Rühren in 3-10 Stunden zugetropft. Nach dem Abkühlen wurde das Reaktionsgemisch mit 200 ml Ether versetzt, mit Eisen(II)-sulfat zur Zerstörung der Peroxide, dann mit Sodalösung und anschließend mit Wasser neutral gewaschen, über Natriumsulfat getrocknet und eingeengt. Der überschüssige Ausgangsalkohol wurde abdestilliert und das Produkt im Ölpumpenvakuum fraktioniert destilliert.

Ausbeuten :

32,7 g 1-Oxa-spiro-[4,7]-dodecan-2-on

$Kp_{0,07}$ 86 °C

$^1$H-NMR ($CDCl_3$) $\delta$ = 2,58 ppm ( 2H, t)
1,7 ppm (16H, m)

$n^{20}_D$ 1,494 9

20,6 g 1-Oxa-4-methyl-spiro-[4,7]-dodecan-2-on

$Kp_{0,02}$ 105 °C

$^1$H-NMR ($CDCl_3$) $\delta$ = 1,9 ppm (17H, m)
1,03 ppm ( 3H, d)

### B. Herstellung der Ether

1,1 mol $LiAlH_4$ wurden in 150 ml Ether unter trockenem Stickstoff vorgelegt. 1 mol Lacton, gelöst in 150 ml Ether, wurde unter Rühren langsam zugetropft und eine Stunde nachgerührt. Unter Kühlung wurden dann langsam 220 ml Wasser zugetropft und mit 10 %iger Schwefelsäure angesäuert. Die organische Phase wurde abgetrennt und mit gesättigter Natriumchloridlösung gewaschen. Der Ether wurde abdestilliert und der Rückstand in Toluol aufgenommen.

Nach Zugabe von 17 g para-Toluol-sulfonsäure wurde zum Sieden erhitzt und das entstehende Reaktionswasser azeotrop abdestilliert. Die Toluollösung wurde neutral gewaschen und eingeengt, der Spiroether fraktioniert destilliert.

Ausbeuten :

90,7 g 1-Oxa-spiro-[4,7]-dodecan (1)

$Kp_{16}$ 103 °C

$^1$H-NMR($CDCl_3$) $\delta$ = 3,72 ppm ( 2H, t)
= 1,80 ppm (18H, m)

$n^{20}_D$ 1,482 8

Geruch : Zibet-, Skatol-, Indol-Note

91,2 g 1-Oxa-4-methyl-spiro-[4,7]-dodecan (2)

$Kp_{20}$ 115 °C

$^1H$-NMR($CDCl_3$) δ = 3,72 ppm ( 2 H, t)
= 1,84 ppm (17 H, m)
= 1,0 ppm ( 3 H, d)

Geruch : erdig, technisch, Indol-Note

C. Riechstoffkomposition

Chypre-Formulierung

| | |
|---|---|
| 1-Oxa-spiro-[4,7]-dodecan | 300 Gew.-Teile |
| Galaxolid | 300 Gew.-Teile |
| Ambroxan® (Henkel) (10 %ig in IPM) | 300 Gew.-Teile |
| Bergamottöl | 50 Gew.-Teile |
| Rosenöl bulgar. | 30 Gew.-Teile |
| Karottensamenöl | 20 Gew.-Teile |
| Selleriesamenöl | 10 Gew.-Teile |
| α-Methyljonon | 10 Gew.-Teile |
| Vetiveröl | 10 Gew.-Teile |
| Jasmin Absolue | 10 Gew.-Teile |
| Atrinon® (Henkel) | 10 Gew.-Teile |
| Sandelholzöl | 5 Gew.-Teile |
| Moschus Keton | 3 Gew.-Teile |
| Orangenöl (terpenfrei) | 5 Gew.-Teile |
| Eichenmoos Absolue | 3 Gew.-Teile |
| Undecylenaldehyd (10 %ig in DEP) | 3 Gew.-Teile |
| Cyclopentadecanolid | 2 Gew.-Teile |
| Angelicawurzelöl | 1 Gew.-Teil |
| Phenylethylalkohol | 28 Gew.-Teile |
| | 1 000 Gew.-Teile |

IPM = Isopropylmyristat
DEP = Diethylphthalat

**Patentansprüche**

1. 1-Oxa-spiro-[4,7]-dodecane der Formeln (1) oder (2)

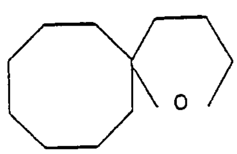

oder

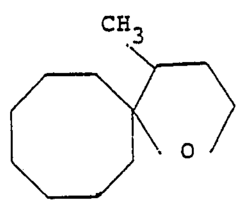

(1) (2)

2. Verfahren zur Herstellung der 1-Oxa-spiro-[4,7]-dodecane der Formeln (1) oder (2) dadurch gekennzeichnet, daß man

a) Acrylsäure oder Crotonsäure an Cyclooctanol radikalisch addiert, wobei die Reaktion in verdünnter Lösung durchgeführt und mit Di-tert.-butylperoxid gestartet wird, und anschließend die intermediär entstehende Hydroxysäure unter diesen Reaktionsbedingungen zum Lacton

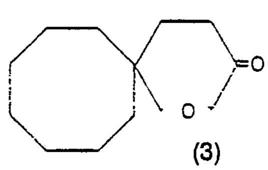

oder

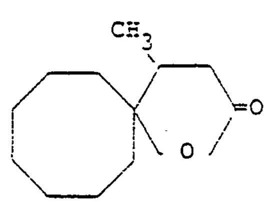

(3)

cyclisiert,

b) das Lacton mit einem komplexen Metallhydrid zum Diol

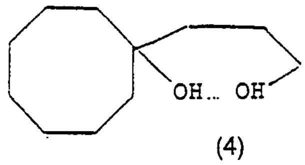 oder 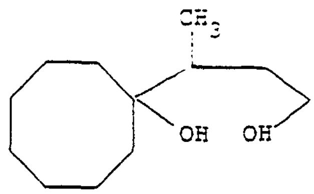

(4)

reduziert, und schließlich das

c) Diol unter Cyclisierung zu (1) oder (2) durch azeotropes Abdestillieren des Wassers mit Toluol unter Verwendung von p-Toluolsulfonsäure in einer Menge von 10 %, bezogen auf das Diol, als Katalysator dehydratisiert.

3. Verwendung von 1-Oxa-spiro-[4,7]-dodecanen der Formeln (1) oder (2) als Riechstoffe.

4. Riechstoffkompositionen, gekennzeichnet durch einen Gehalt an 1-Oxa-spiro-[4,7]-dodecanen der Formeln (1) oder (2).

**Claims**

1. 1-Oxaspiro-[4,7]-dodecanes corresponding to formula (1) or (2) below

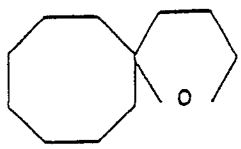 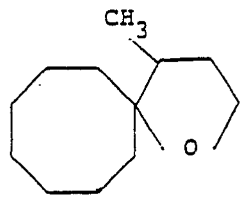

(1) (2)

2. A process for the preparation of the 1-oxaspiro-[4,7]-dodecanes corresponding to formula (1) or (2), characterized in that

a) acrylic acid or crotonic acid is subjected to radical addition with cyclo-octanol, the reaction being carried out in dilute solution and being initiated by di-tert.-butyl peroxide, after which the hydroxy acid formed as intermediate is cyclized under these reaction conditions to the lactone

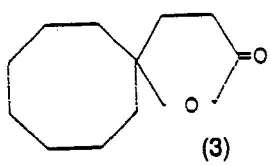 or 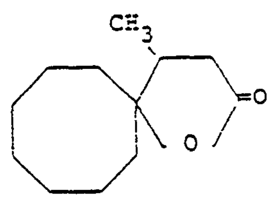

(3)

b) the lactone is reduced with a complex metal hydride to the diol

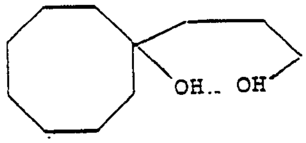 or 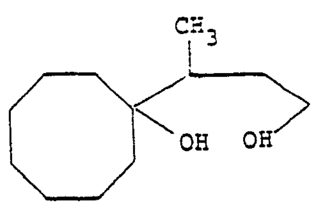

and finally

c) the diol is dehydrated with cyclization to (1) or (2) by azeotropic distillation of the water with toluene using p-toluene sulfonic acid in a quantity of 10 %, based on the diol, as catalyst.

3. The use of 1-oxaspiro-[4,7]-dodecanes corresponding to formula (1) or (2) as perfumes.

4. Perfume compositions containing 1-oxaspiro-[4,7]-dodecanes corresponding to formula (1) or (2).

**Revendications**

1. 1-oxa-spiro-[4,7]-dodécane de formule (1) ou (2) :

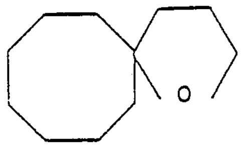

(1)

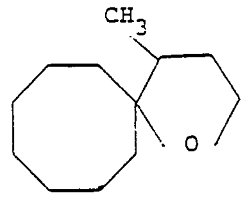

(2)

2. Procédé d'obtention des 1-oxa-spiro-[4,7]-dodécane de formule (1) ou (2) caractérisé en ce qu'on additionne par une réaction radicalaire

a) l'acide acrylique ou l'acide crotonique ou Cyclooctanol dans laquelle la réaction est effectuée en solution diluée et démarrée avec du peroxyde de di-terbutyle, et immédiatement l'hydroxyacide formé intermédiairement dans ces conditions réactionnelles est cyclisé en lactone :

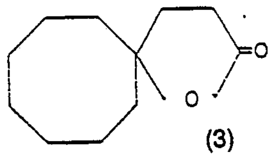

(3)

ou

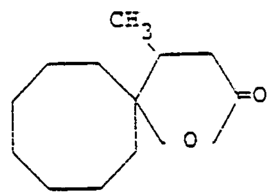

b) la lactone est réduite en diol à l'aide d'un hydrure métallique complexe :

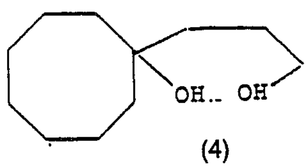

(4)

ou

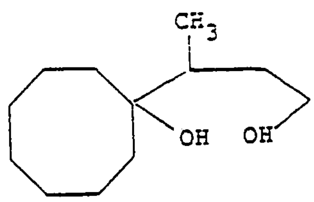

et finalement

c) le diol est déshydraté avec cyclisation en (1) ou en (2) par distillation azéotropique de l'eau au moyen de toluène en utilisant l'acide p-toluène sulfonique en quantité de 10 % par rapport au Diol, comme catalyseur.

3. Utilisation des 1-oxa-spiro-[4,7]-dodécane de formule (1) ou de formule (2) comme parfums.

4. Compositions de parfum caractérisées par une teneur en 1-oxa-spiro-[4,7]-dodécanes de formule (1) ou de formule (2).